Europäisches Patentamt

**European Patent Office**

Office européen des brevets

(19)

(11) Publication number: **0 107 058**
**B1**

(12) **EUROPEAN PATENT SPECIFICATION**

(45) Date of publication of patent specification: **02.09.87**

(51) Int. Cl.⁴: **G 08 B 21/00, A 61 B 5/10**

(21) Application number: **83109524.5**

(22) Date of filing: **24.09.83**

(54) Apparatus for monitoring the presence of a person in a bed.

(30) Priority: **30.09.82 US 429047**
**04.05.83 US 491355**

(43) Date of publication of application:
**02.05.84 Bulletin 84/18**

(45) Publication of the grant of the patent:
**02.09.87 Bulletin 87/36**

(84) Designated Contracting States:
**AT BE CH DE FR GB IT LI LU NL SE**

(56) References cited:
**DE-A-3 033 858**
**US-A-4 179 692**
**US-A-4 228 426**
**US-A-4 264 904**
**US-A-4 295 133**

(73) Proprietor: **Bed-Check Corporation**
**P.O. Box 170**
**Tulsa Oklahoma 74103 (US)**

(72) Inventor: **Musick, Jeff L.**
**4042 East 52 Place**
**Tulsa Oklahoma 74135 (US)**
Inventor: **Blaker, David G., Jr.**
**1354 East 26 Street**
**Tulsa Oklahoma 74114 (US)**
Inventor: **Blaker, Robert D.**
**1502 South Boulder, No. 9C**
**Tulsa Oklahoma 74119 (US)**
Inventor: **Vance, Dwight A.**
**101 South Joshua**
**Broken Arrow Oklahoma 74102 (US)**

(74) Representative: **Cole, David John**
**Executive Liaison Services 54 Templemere**
**Weybridge Surrey KT13 9PB (GB)**

Courier Press, Leamington Spa, England.

## Description

This invention relates to an apparatus for monitoring the presence of a person in a bed. The apparatus is primarily intended for monitoring the presence or absence of a hospital patient from a bed, but may obviously be used for monitoring persons in environments other than hospitals.

A problem continually encountered in hospitals is that of patients leaving their beds when the patients are not in a condition such that they can safely be trusted out of bed. Many instances have been reported of elderly or infirm patients leaving hospital beds and falling, breaking bones or otherwise causing injury to themselves. The patients are sometimes disoriented and confused because of the unfamiliar hospital environment or the effect of drugs used for therapeutic purposes, and such disorientation or confusion can cause the patients to wander, the patients even sometimes leaving the hospital altogether. For these reasons, it is important that hospital staff be apprised if a patient leaves a hospital bed, and several types of prior art monitoring devices for this purpose have previously been developed.

In order for such monitoring equipment to function properly in order to indicate that a patient has left his or her bed, a convenient, reliable switch mechanism must be provided to open and close an electrical circuit. This invention seeks to provide such a switch mechanism.

The document DE—A—30 33 858 describes a sensor element for use in a security system. This sensor element comprises a thin base member having a large number of small apertures passing therethrough, and two electrically conductive members disposed on opposed sides of the base member, the base member and electrically conductive members being enclosed by two insulating cover members. The two electrically conductive members are normally held apart by the base member, but when the sensor element is placed beneath an object to be secured (the drawings of the mentioned document show it being used to protect a typewriter), the weight of the object resting on the sensor element distorts it, thereby enabling the electrically conductive members to contact one another via the apertures in the base member.

The document US—A—4,264,904 describes a bed mat for use in detecting the presence of a patient in a bed. This sensing mat is divided into a number of parallel compartments, each of which is provided with a pair of metal strips which are normally held apart but which contact one another when a patient rests upon the appropriate compartment. The pairs of strips are wired in parallel so that, when the patient is in the bed, a circuit connected to the strips is closed by the contact between at least one pair of strips.

The present invention provides a switching apparatus which can detect the presence of a patient in a bed, but which does not need to cover the whole bed.

This invention provides a switching apparatus capable of being placed in a bed, this apparatus comprising:

an elongate, thin, generally rectangular base member formed of insulating flexible material and having a pair of surfaces on opposed sides thereof and an aperture passing through the base member and the pair of surfaces;

a first thin, electrically conductive member having dimensions not substantially greater than those of the base member and having a pair of surfaces on opposed sides thereof, one of the surfaces on the first conductive member being supported upon the upper one of the pair of surfaces on the base member;

a second thin, electrically conductive member having dimensions not substantially greater than those of the base member and having a pair of surfaces on opposed sides thereof, one of the surfaces on the second conductive member being supported upon the lower one of the pair of surfaces on the base member;

a first non-conductive, thin, generally rectangular plastic cover member affixed to the other of the pair of surfaces on the first conductive member;

a second non-conductive, thin, generally rectangular plastic cover member affixed to the other of the pair of surfaces on the second conductive member; and

a cable having first and second conductors, the first conductor being connected to the first conductive member and the second conductor being connected to the second conductive member,

the first and second cover members being of width and length greater than the base member and providing edge portions overlapping the edges of the base member, the edge portions of the first and second cover members being sealed to one another, and

the first and second conductive members being normally supported by the base member in spaced-apart relationship by the memory of the base member and the plastic cover members but contacting one another when the apparatus is subjected to distortion.

The apparatus of the present invention is characterized in that the base member has a length of 635 to 1016 mm, a width of 95.3 to 127 mm and a thickness of substantially 1.6 mm, the base member has only a single aperture passing therethrough, this aperture having a length of 571 to 965 mm and a width of 45 to 57 mm; and the end portion of the cable adjacent the points at which the first and second conductors are connected to the first and second conductive members is positioned between the first and second cover members.

The switching device of the invention is intended to be placed in the bed to be monitored underneath the person therein so that when the person is lying in the bed the base member will be deformed, thus allowing the two conductive members to contact one another via the aperture in the base member and thereby establishing electrical contact between the two conductors.

(All references herein to "conductive" and "insulating" and words of similar import refer to electrical conduction or insulation.) Thus, while the person is lying down on the bed on the switching device, electrical contact will exist between the two conductors but when the person is out of the bed the switching device will be open. Obviously, the switching device is in the form of a thin mat having a thickness much less than its horizontal dimensions; not only does the provision of this type of switching device increase the comfort of the person being monitored, but provided the mat is appropriately sized and appropriately located in the bed the switching device will be open when the person is off the sensing device even if the person is still sitting on the edge of the bed and it will thus be possible to detect the person leaving the bed before the person wanders from the immediate vicinity thereof.

It is desirable that the aperture in the base member be centrally positioned. An appropriate insulating flexible material for forming the base member is high-density polyethylene foam.

As already mentioned, in the switching device of the invention, two thin, electrically conductive members are affixed to the opposed surfaces of the base member. These electrically conductive members may have the form of metal foils, metal foils backed with plastic or metalized plastic material (such metalized plastic material may be produced, for example, by applying electroconductive ink to a polyester or other film) and are conveniently secured to the base member by adhesive. Thus, when the base member is in its undeformed state, it supports the conductive members spaced apart from one another by a distance approximately equal to the thickness of the base member. Two conductors are electrically conducted one to each conductive member; conveniently, the two connectors are brought together within a single cable. The cover members, which are conveniently made of plastic serve to cover the conductive members and base member; the sealing of the peripheral portions of the cover members to one another serves to completely seal the apparatus so that the person being monitored is not exposed to electrical shocks from the conductors or the conductive members when the apparatus is placed in the bed.

The switching device of the invention can be placed in any convenient position beneath the person in the bed; thus, for example, the switching device can be placed beneath sheets, bedding or similar covers positioned on top of the mattress, and the switching device need not come into direct contact with the person when the person leaves the bed, the rigidity of the device, and particularly the resilience of the flexible base member, sufficient to cause the conductive members to separate from one another, thereby opening the switching device to indicate the absence of the person from the bed.

Preferred embodiments of the invention will now be described, though by way of illustration only, with reference to the accompanying drawings in which:

Fig. 1 is a top plan view of a preferred switching device of the invention;

Fig. 2 is an enlarged top plan view of part of the switching device shown in Fig. 1 with various parts broken away to show the internal construction of the switching device; and

Fig. 3 is a cross section taken across the width of the device shown in Figs. 1 and 2 (i.e. vertically in Fig. 1).

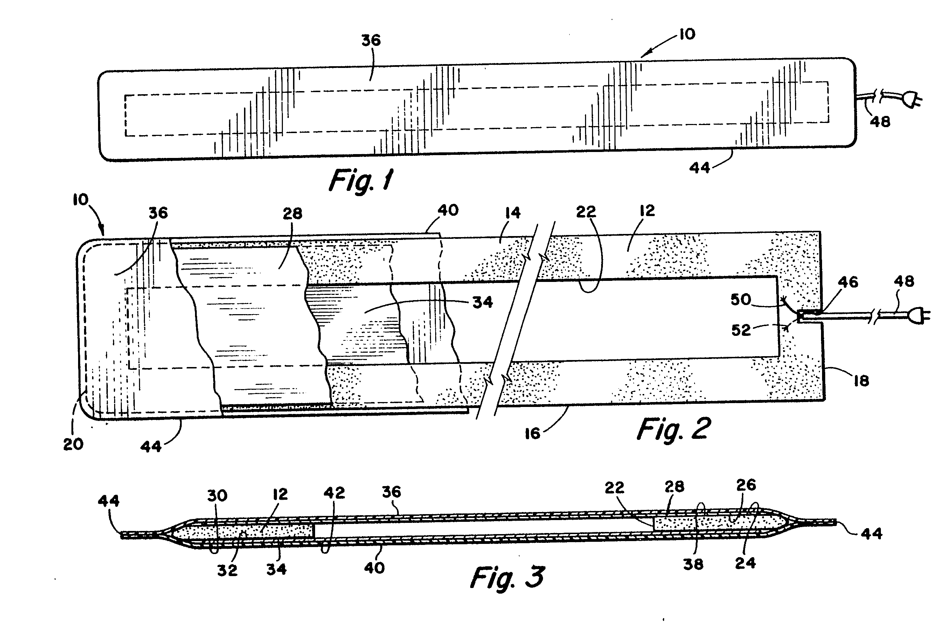

As shown in Figs. 1—3, the preferred switching device of the invention (generally designated 10) comprises a thin base member 12 which is formed of insulating flexible material, conveniently high-density polyethylene foam. The base member 12 is generally rectangular having opposed side edges 14 and 16 and ends 18 and 20. A rectangular aperture 22 is cut centrally through the base member 12, the dimensions of the aperture 22 being approximately 38 to 64 mm smaller in both width and length than those of the base member 12 itself. The base member of the preferred switching device shown in Figs. 1—3 has a length from end 18 to end 20 of approximately 635 to 1016 mm while its width between the side edges 14 and 16 is 95.3 to 127 mm. The thickness of the base member measured between its surface 24 and its lower surface 30 is approximately 1.6 mm. The aperture 22 is conveniently 45 to 57 mm wide and 571 to 965 mm long.

As best seen in Figs. 2 and 3, the base member 12 is sandwiched between two thin, electrically conductive members 28 and 34, which are both slightly smaller in both width and length than the base member 12 itself. The lower surface 26 of the conductive member 28 is adhesively affixed to the upper surface 24 of the base member 12, while the upper surface 32 of the conductive member 34 is adhesively affixed to the lower surface 30 of the base member 12. In the preferred switching device shown in Figs. 1—3, the conductive members 28 and 34 are made of thin metal, such as tin foil, aluminum foil or a similar metal surface, but alternatively the conductive members may be made of metal foils bonded to a plastic backing sheet or may be formed of metalized plastic, produced for example by applying electroconductive ink to a plastic foil.

The base member 12 and the conductive members 28 and 24 are all sandwiched between two plastic cover members 36 and 40. As shown in Figs. 2 and 3, the upper cover member 36 has its lower surface bonded to the upper surface 38 of the conductive member 28, while the upper surface of the lower cover member 40 is bonded to the lower surface 42 of the conductive member 34. The length and width of the cover members 36 and 40 are both slightly greater than the corresponding dimensions of the base member 12 so that the peripheral portions 44 of the cover members 36 and 40 extend outwardly beyond the periphery of the conductive members 28 and 34 and the base member 12. These peripheral portions 44 of the two cover members are sealed to one another so that no portions of the base member 12 or the conductive members 28 and 34 are exposed.

To provide external connections to the conductive members 28 and 34, a notch 46 is cut in the end 18 of the base member 12 and a cable 48 extends between the peripheral portions 44 of the cover members 36 and 40 into this notch 46. The cable 48 carries a first conductor 50 which is connected, between the cover members 36 and 40, to the conductive member 28, and a second conductor 52 which is similarly connected, between the cover members 36 and 40 to the second conductive member 34.

The preferred switching device of the invention shown in Figs. 1—3 may be easily assembled in the following manner. The base member 12 has the notch 46 cut therein and adhesive is then applied to both the top and bottom surfaces 24 and 30 of the base member. The cable 48 is next inserted into the notch 46 and the conductors 50 and 52 exposed so as to lie adjacent the upper and lower surfaces 24 and 30 respectively of the base member 12. The conductive members 28 and 34 are then positioned on the base member, thereby engaging the connectors 50 and 52 respectively. The cover members 36 and 40 are then placed in the appropriate positions in contact with the conductive members 28 and 34 respectively, adhesive being placed between each cover member and the adjacent surface of the conductive member. The assembled switching device is then pressed together by means of a press or rollers so as to bond the various members together and the peripheral portions 44 of the cover members 36 and 40 are sealed to each other to complete the assembly of the switching device. It will be seen that all the elements used in the switching device of the invention are inexpensive and that the switching device can be expeditiously assembled. Thus, the switching device can be manufactured sufficiently cheaply to be considered a disposable item and its use limited to a single patient, although if desired the device can be sterilized for reuse.

As will be apparent to those skilled in this field, the preferred switching device shown in Figs. 1—3 operates as follows. When the base member is in its undeformed state, as shown in Fig. 3, the flexible base member holds the conductive members 28 and 34 spaced from one another by a distance substantially equal to the thickness of the base member. Thus, in this condition, there is no electrical contact between the conductors 50 and 52. However, when a person lies, sits or otherwise disposes himself upon the switching device, the weight of the person distorts the switching device sufficiently to cause the conductive members 28 and 34 to come into contact with one another via the aperture 22 in the base member 12 at one or more places, thus providing electrical contact between the conductors 50 and 52.

**Claim**

Switching apparatus (10) capable of being placed in a bed, the apparatus (10) comprising:

an elongate, thin, generally rectangular base member (12) formed of insulating flexible material and having a pair of surfaces (24, 30) on opposed sides thereof and an aperture (22) passing through the base member (12) and through the pair of surfaces (24, 30);

a first thin, electrically conductive member (28) having dimensions not substantially greater than those of the base member (12) and having a pair of surfaces (26, 32) on opposed sides thereof, one of the surfaces (26) on the first conductive member (28) being supported upon the upper one (24) of the pair of surfaces on the base member (12);

a second thin, electrically conductive member (34) having dimensions not substantially greater than those of the base member (12) and having a pair of surfaces (38, 42) on opposed sides thereof, one of the surfaces (38) on the second conductive member (34) being supported on the lower one (30) of the pair of surfaces on the base member (12);

a first non-conductive, thin, generally rectangular plastic cover member (36) affixed to the other (32) of the pair of surfaces on the first conductive member (28);

a second non-conductive, thin, generally rectangular plastic cover member (40) affixed to the other (42) of the pair of surfaces on the second conductive member (34); and

a cable (48) having first and second conductors (50, 52), the first conductor (50) being connected to the first conductive member (28) and the second conductor (52) being connected to the second conductive member (34);

the first and second cover members (36, 40) being of length and width greater than the base member (12) and providing edge portions overlapping the edges of the base member (12), the edge portions of the first and second cover members (36, 40) being sealed to one another,

the first and second conductive members (28, 34) being normally supported by the base member (12) in spaced-apart relationship by the memory of the base member (12) and the plastic cover members (36, 40) but contacting one another when the apparatus is subjected to distortion

characterized in that:

the base member (12) has a length of 635 to 1016 mm, a width of 95.3 to 127 mm and a thickness of substantially 1.6 mm, the base member (12) having a single aperture (22) passing therethrough, this aperture (22) having a length of 571 to 965 mm and a width of 45 to 57 mm; and

the end portion of the cable (48) adjacent the points at which the first and second conductors (50, 52) are connected to the first and second conductive members (28, 34) respectively being positioned between the first and second cover members (36, 40).

## Patentanspruch

1. Schaltapparat (10), welcher in einem Bett angeordnet werden kann, bestehend aus

einem langgestreckten dünnen, im allgemeinen rechtwinkeligen Basisteil (12) aus biegsamen Isoliermaterial mit einem Paar Fläche (24, 30) auf seinen gegenüberliegenden Seiten und mit einer Öffnung (22), die durch den Basisteil (12) und das Flächenpaar (24, 30) hindurchgeht,

einem ersten dünnen, elektrisch leitenden Teil (28), dessen Abmessungen nicht wesentlichen größer sind als diejenigen des Basisteiles (12) und der ein Paar Flächen (26, 32) an seinen gegenüberliegenden Seiten aufweist, wobei die eine Fläche (26) des ersten leitenden Teiles (28) auf der oberen Fläche (24) der beiden Flächen des Basisteiles (12) aufliegt,

einem zweiten dünnen, elektrisch leitenden Teil (34), dessen Abmessungen nicht wesentlich größer sind als diejenigen des Basisteiles (12) und der ein Paar Flächen (38, 42) an seinen gegenüberliegenden Seiten aufweist, wobei die eine Fläche (38) des zweiten leitenden Teiles (34) auf der unteren Fläche (30) der beiden Flächen des Basisteiles (12) aufliegt,

einem ersten, nicht leitenden, dünnen, im allgemeinen rechteckigen Plastik-Abdeckteil (36), der an der anderen Fläche (32) der beiden Flächen des ersten leitenden Teiles (28) befestigt ist,

einem zweiten, nicht leitenden, dünnen, im allgemeinen rechteckigen Plastik-Abdeckteil (40), der an der anderen Fläche (42) der beiden Flächen des zweiten leitenden Teiles (34) befestigt ist, und

einem Kabel (48) mit einem ersten und einem zweiten Leiter (50, 52), wobei der erste Leiter (50) mit dem ersten leitenden Teil (28) und der zweite Leiter (52) mit dem zweiten leitenden Teil (34) verbunden ist,

wobei der erste und zweite Abdeckteil (36, 40) in Länge und Breite größer als der Basisteil (12) sind und Randbereiche aufweisen, welche die Ränder des Basisteiles (12) überlappen, und wobei die Randbereiche des ersten und zweiten Abdeckteiles (36, 40) dichtend miteinander verbunden sind,

wobei der erste und zweite Leiterteil (28, 34) im Normalzustand vom Basisteil (12) infolge des Formerinnerungsvermögens des Basisteiles (12) und der Plastik-Abdeckteile (36, 40) in Abstand voneinander gehalten sind, sich aber berühren, wenn der Apparat einer Verformung ausgesetzt wird, dadurch gekennzeichnet, dass

der Basisteil (12) eine Länge von 635 bis 1016 mm, eine Breite von 95,3 bis 127 mm und eine Dicke von etwa 1,6 mm aufweist, wobei der Basisteil (12) eine einzige hindurchgehende Öffnung (22) mit einer Länge von 571 vis 965 mm und einer Breite von 45 bis 57 mm aufweist und

dass der Endabschnitt des Kabels (48) nahe den Punkten, an denen der erste und zweite Leiter (50, 52) mit dem ersten bzw. zweiten leitenden Teil (28, 34) verbunden sind, zwischen dem ersten und dem zweiten Abdeckteil (36, 40) angeordnet ist.

## Revendication

Dispositif de commutation (10) susceptible d'être placé dans un lit, le dispositif (10) comprenant:

— un organe de base (12) généralement rectangulaire, mince et allongé, formé en un matériau flexible isolant, et ayant une paire de surfaces (24, 30) sur ses côtés opposés et une ouverture (22) traversant l'organe de base (12) et la paire de surfaces (24, 30);

— un premier organe électriquement conducteur mince (28) ayant des dimensions qui ne sont pas sensiblement plus grandes que celles de l'organe de base (12) et ayant une paire de surfaces (26, 32) sur ses côtés opposés, une des surfaces (26) sur le premier organe conducteur (28) étant supportée sur la surface supérieure (24) de la paire de surfaces sur l'organe de base (12);

— un second organe électriquement conducteur mince (34) ayant des dimensions qui ne sont pas sensiblement plus grandes que celles de l'organe de base (12), et ayant une paire de surfaces (38, 42) sur ses côtés opposés, une des surfaces (38) sur le second organe conducteur (34) étant supportée sur la surface inférieure (30) de la paire de surfaces sur l'organe de base (12);

— un premier organe de recouvrement en matière plastique généralement rectangulaire, non conducteur, mince (36), fixé à l'autre surface (32) de la paire de surfaces sur le premier organe conducteur (28);

— un second organe de recouvrement en matière plastique généralement rectangulaire, mince, non conducteur (40), fixé à l'autre surface (42) de la paire de surfaces sur le second organe conducteur (34); et

— un câble (48) ayant des premier et second conducteurs (50, 52), le premier conducteur (50) étant relié au premier organe conducteur (28) et le second conducteur (52) étant relié au second organe conducteur (34),

— les premier et second organes de recouvrement (36, 40) ayant une longueur et une largeur plus grandes que l'organe de base (12) et fournissant des parties de bord recouvrant les bords de l'organe de base (12), les parties de bord des premier et second organes de recouvrement (36, 40) étant scellées l'une à l'autre,

— les premier et second organes conducteurs (28, 34) étant normalement supportés par l'organe de base (12) en relation espacée par le mémoire de l'organe de base (12) et les organes de recouvrement en matière plastique (36, 40), mais venant en contact l'un de l'autre quand le dispositif est soumis à une déformation, caractérisé en ce que:

— l'organe de base (12) a une longueur de 635 à 1016 mm, une largeur de 95,3 à 127 mm et une épaisseur de sensiblement 1,6 mm, l'organe de base (12) ayant une seule ouverture (22) le traversant, cette ouverture (22) ayant une longueur de 571 à 965 mm et une largeur de 45 à 57 mm; et

— la partie d'extrémité du câble (48) adjacente aux points auxquels les premier et second conducteurs (50, 52) sont reliés aux premier et second organes conducteurs (28, 34) respectivement étant placée entre les premier et second organes de recouvrement (36, 40).

Fig. 1

Fig. 2

Fig. 3

0 107 058